# EUROPEAN PATENT APPLICATION

(11) **EP 4 699 648 A1**
(43) Date of publication of application: **25.02.2026**
(21) Application number: 24195459.3
(22) Date of filing: 20.08.2024
(51) Int. Cl.: A61N 5/10, G16H 20/40, G16H 70/00

(54) **FACILITATING PATIENT TREATMENT PLANNING**

(71) Applicant: Siemens Healthineers International AG, 6312 Steinhausen (CH)
(72) Inventor: LAAKSONEN, Hannu, 02130 Espoo (FI); CORDERO MARCOS, Maria, 02620 Espoo (FI); HAKALA, Mikko, 05200 Rajamaki (FI)
(74) Representative: Mathisen & Macara LLP

(57) **Abstract**

A system for facilitating patient treatment planning includes a first interface (102) configured to communicate with a patient data source (104) to receive data relating to a patient and a second interface (106) configured to communicate with a treatment planning tool data source to receive data relating to one or more treatment planning tools (108). A processor (110) is coupled to the first interface (102) and the second interface (106), and configured to determine whether the data relating to the patient matches that required by one or more of the treatment planning tools (108) and, if the data relating a patient matches that required by one or more of the tools (108), to automatically initiate an action.

## Description

### TECHNCAL FIELD

This invention relates to a system for and method of facilitating patient treatment planning.

### BACKGROUND

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

Treatment plans for radiation treatment sessions are often automatically generated through a so-called optimisation process. As used herein, "optimisation" will be understood to refer to improving a candidate treatment plan without necessarily ensuring that the optimised result is, in fact, the singular best solution. Such optimisation often includes automatically adjusting one or more physical treatment machine parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In some cases, optimisation proceeds as a function of multiple different criteria. Various prediction and descriptive models are known to optimise treatment planning. Artificial intelligence (Al)/machine learning may be used to optimise treatment planning.

The main operational mode of present-day optimisation tools, including Al/machine learning models, in healthcare is still essentially isolated usage of models and results, without integration into a broader healthcare subsystem. Whenever an optimisation tool is applied, the interfaces are typically specified separately in each product.

### SUMMARY OF THE INVENTION

According to a first aspect of the invention, there is provided system for facilitating patient treatment planning as defined by claim 1.

Optional features are defined by the dependent claims.

According to the first aspect of the invention, there is provided a system for facilitating patient treatment planning, the system including: a first interface configured to communicate with a patient data source to receive data relating to a patient; a second interface configured to communicate with a treatment planning tool data source to receive data relating to a plurality of treatment planning tools; a processor coupled to the first interface and the second interface, and configured to determine whether the data relating to the patient matches that required by one or more of the treatment planning tools and, if the data relating a patient matches that required by one or more of the tools, to automatically initiate an action.

The action may include using the treatment planning tools to perform segmentation, beam placement or automatic radiotherapy planning based on the data relating to the patient. The action may include using the treatment planning tools to predict an outcome of a treatment planned by the treatment planning tools planning based the data relating to the patient, such as survivability and/or side effects. The automatic initiation of the action allows tools to be used as soon as the data required thereby becomes available.

The treatment planning tool may comprise an analytics tool. The treatment planning tool may comprise a treatment planning model, a prediction model or descriptive model. The tool may use Al/machine learning.

The second interface may be configured to maintain a plurality of second interface data fields relating to respective treatment planning tool characteristics, each second interface data field having a predetermined format, and to maintain a record for each treatment planning tool comprising ones of the second interface data fields derivable from the received data relating the treatment planning tools. This may allow different formats of data used by/received from respective treatment planning tools to be stored in the interface in a consistent format, e.g. by converting the different formats into a standard format for storage by the interface.

The second interface data fields may be meta data fields.

The second interface data fields may include one or more of name, version, data input requirements, treatment planning tool data output type and serving method.

The processor may be configured such that determining whether the data relating to the patient matches that required by one or more of the treatment planning tools includes analysing data from the records in the second interface corresponding to the one or more of the treatment planning tools. If the data are stored in a standard format this may advantageously make this analysis more efficient and effective for multiple tools.

The action may include executing the one or more of the treatment planning tools to produce treatment planning tool data output corresponding to the data relating to the patient. The data output may be a treatment plan.

The action may include prompting a user prior to executing the one or more of the treatment planning tools to produce treatment planning tool data output corresponding to the data relating to the patient.

The action may include listing the treatment planning tools ready for execution.

The first interface may be configured to maintain a plurality of first interface data fields relating to respective patient characteristics, each first interface data field having a predetermined format, and to maintain a record for each patient comprising ones of the first interface data fields derivable from the received data relating to the patient. This may allow different formats of data relating to respective patients tools to be stored in the interface in a consistent format, e.g. by converting the different formats into a standard format for storage by the interface.

The first interface data fields may be meta data fields.

The first interface data fields include one or more of patient information, results from health treatments, patient images and multiomics information.

The processor may be configured such that determining whether the data relating to a patient matches that required by one or more of the treatment planning tools includes analysing data from the records in the first interface corresponding to the patient. If the data relating to the patient are stored in a standard format this may advantageously make this analysis more efficient and effective for multiple patients.

The processor may be configured to determine whether the data relating to a patient does not match that required by one or more of the treatment planning tools and, if the data relating a patient does not match that required by one or more of the treatment planning tools, to identify further data needed by the one or more of the treatment planning tools. The processor may be configured to indicate to the further data needed by the one or more of the treatment planning tools. The processor may be configured to indicate to the further data needed by the one or more of the treatment planning tools in priority order.

The processor may be configured to automatically trigger processing by one of the treatment planning tools to obtain the further data needed by the one or more of the treatment planning tools.

The processor may be configured to authenticate users for selectively making available output of the system in dependence upon a user's access rights. This may enhance safety and security of the system.

The processor may be configured to aggregate treatment planning tool output data from a plurality of the treatment planning tools - e.g. by storing output data from the plurality of the treatment planning tools in a predetermined format.

The processor may be configured to receive treatment planning tool output data from one of the treatment planning tools and to determine whether the treatment planning tool output data therefrom matches that required by one or more of the other treatment planning tools and, if the treatment planning tool output data matches that required by one or more of the other treatment planning tools, to automatically execute a further action.

The further action may include (automatically) executing the one or more of the other treatment planning tools using the treatment planning tool output data from the one of the treatment planning tools. When the output data from one of the treatment planning tools completes the data required by another one of the treatment planning tools, that other one of the treatment planning tools may advantageously be automatically executed. In this way, the system may automatically build a database of treatment planning tool results with the execution of one tool producing an output that enables the execution of another.

The processor may be configured to create and display on a graphical user interface patient treatment information for a patient including at least one of patient characteristics, treatment planning tool output for the patient, suggested further treatment planning workflows and suggested further data relating to the patient required.

According to a second aspect of the invention, there is provided a method of facilitating patient treatment planning, the method including: providing a first interface configured to communicate with a patient data source to receive data relating to a patient; providing a second interface configured to communicate with a treatment planning tool data source to receive data relating to a plurality of treatment planning tools; and using a processor coupled to the first interface and the second interface to determine whether the data relating to the patient matches that required by one or more of the treatment planning tools and, if the data relating a patient matches that required by one or more of the tools, automatically initiating an action.

The action may include using the treatment planning tools to perform segmentation, beam placement or automatic radiotherapy planning based on the data relating to the patient. The action may include using the treatment planning tools to predict an outcome of a treatment planned by the treatment planning tools planning based the data relating to the patient, such as survivability and/or side effects. The automatic initiation of the action allows tools to be used as soon as the data required thereby becomes available.

The treatment planning tool may be an analytics tool. The treatment planning tool may be a treatment planning model, a prediction model or descriptive model. The tool may use Al/machine learning.

The second interface may maintain a plurality of second interface data fields relating to respective treatment planning tool characteristics, each second interface data field having a predetermined format, and may maintain a record for each treatment planning tool comprising ones of the second interface data fields derivable from the received data relating the treatment planning tools. This may allow different formats of data used by/received from respective treatment planning tools to be stored in the interface is a consistent format, e.g. by converting the different formats into a standard format for storage by the interface.

The second interface data fields may be meta data fields.

The second interface data fields may include one or more of name, version, data input requirements, treatment planning tool data output type and serving method.

The processor may be operated such that determining whether the data relating to the patient matches that required by one or more of the treatment planning tools includes analysing data from the records in the second interface corresponding to the one or more of the treatment planning tools. If the data are stored in a standard format this may advantageously make this analysis more efficient and effective for multiple tools.

The action may include executing the one or more of the treatment planning tools to produce treatment planning tool data output corresponding to the data relating to the patient. The data output may be a treatment plan.

The action may include prompting a user prior to executing the one or more of the treatment planning tools to produce treatment planning tool data output corresponding to the data relating to the patient.

The action may include listing the treatment planning tools ready for execution.

The first interface may maintain a plurality of first interface data fields relating to respective patient characteristics, each first interface data field having a predetermined format, and may maintain a record for each patient comprising ones of the first interface data fields derivable from the received data relating to the patient. This may allow different formats of data relating to respective patient tools to be stored in the interface is a consistent format, e.g. by converting the different formats into a standard format for storage by the interface.

The first interface data fields may be meta data fields.

The first interface data fields include one or more of patient information, results from health treatments, patient images and multiomics information.

The processor may be operated such that determining whether the data relating to a patient matches that required by one or more of the treatment planning tools includes analysing data from the records in the first interface corresponding to the patient. If the data relating to the patient are stored in a standard format this may advantageously make this analysis more efficient and effective for multiple patients.

The processor may be operated to determine whether the data relating to a patient does not match that required by one or more of the treatment planning tools and, if the data relating a patient does not match that required by one or more of the treatment planning tools, to identify further data needed by the one or more of the treatment planning tools. The processor may be operated to indicate to the further data needed by the one or more of the treatment planning tools. The processor may be operated to indicate to the further data needed by the one or more of the treatment planning tools in priority order.

The processor may be operated to automatically trigger processing by one of the treatment planning tools to obtain the further data needed by the one or more of the treatment planning tools.

The processor may be operated to authenticate users for selectively making available output of the system in dependence upon a user's access rights. This may enhance safety and security of the system.

The processor may be operated to aggregate treatment planning tool output data from a plurality of the treatment planning tools - e.g. by storing output data from the plurality of the treatment planning tools in a predetermined format.

The processor may be operated to receive treatment planning tool output data from one of the treatment planning tools and to determine whether the treatment planning tool output data therefrom matches that required by one or more of the other treatment planning tools and, if the treatment planning tool output data matches that required by one or more of the other treatment planning tools, to automatically execute a further action.

The further action may include (automatically) executing the one or more of the other treatment planning tools using the treatment planning tool output data from the one of the treatment planning tools. When the output data from one of the treatment planning tools completes the data required by another one of the treatment planning tools, that other one of the treatment planning tools may advantageously be automatically executed. In this way, the system may automatically build a database of treatment planning tool results with the execution of one tool producing an output that enables the execution of another.

The processor may be operated to create and display on a graphical user interface patient treatment information for a patient including at least one of patient characteristics, treatment planning tool output for the patient, suggested further treatment planning workflows and suggested further data relating to the patient required.

According to a third aspect of the invention, there is provided a system for facilitating patient treatment planning, the system including: means for communicating with a patient data source to receive data relating to a patient; means for communicating with a treatment planning tool data source to receive data relating to a plurality of treatment planning tools; and processing means for determining whether the data relating to the patient matches that required by one or more of the treatment planning tools and, if the data relating a patient matches that required by one or more of the tools, for automatically initiating an action.

Some embodiments of the invention may provide the ability to gather insights and outcome predictions for a patient before any treatment is done. Embodiments may launch several machine learning or more traditional prediction and descriptive models, and aggregate the results in a meaningful way.

An embodiment may provide a system that serves as a patient hub, which may contain selectively relevant patient information and results from health treatments and related model predictions. Selected information may be provided to authorised stakeholders, who have various access rights in the hub.

The hub of the embodiment may solve the problem of offering in a coherent, organised way to handle requests, retrievals and storage services related to patient and treatment planning model records. Such a hub may seamlessly integrate into a larger healthcare ecosystem.

Embodiments may provide a flexible interface structure that can handle various input and output sources. The hub may handle interfacing with flexible metadata descriptions and can collect the results or launch correct software as needed.

### BRIEF DESCRIPTION OF THE DRAWINGS

For a better understanding of the present disclosure, embodiments will now be described by way of example with reference to the accompanying drawings, in which:
FIG. 1 is an illustration of an example radiotherapy treatment system;
FIG. 2 is a schematic diagram of a system for interfacing between patient records and treatment planning tools such as Al/machine learning models;
FIG. 3 shows a patient interface of the system of FIG. 2 in more detail;
FIG. 4 shows a treatment planning tool interface of the system of FIG. 2 in more detail;
FIG. 5 illustrates examples of planning tool metadata descriptions;
FIG. 6 is a flowchart of steps to facilitate treatment planning;
FIG. 7 is a flowchart of steps to execute a treatment planning tool;
FIG. 8 shows an example GUI generated by the system of FIG. 2; and
FIG. 9 shows a typical computer system upon which the present method may be implemented.

In the drawings like elements are generally designated with the same reference signs.

### DETAILED DESCRIPTION

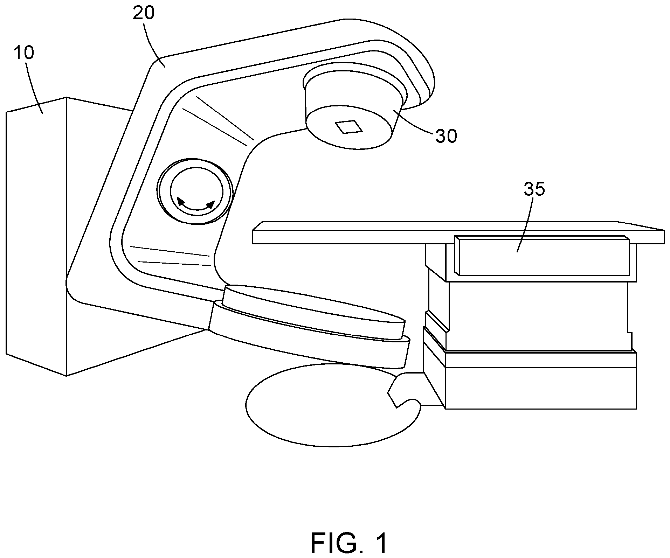

FIG. 1 depicts a radiation treatment system of the type that may be used in to implement a treatment plan. Referring to FIG. 1, a perspective view of a radiation treatment system (in this case a linear accelerator) is shown. Typically, such a system is capable of generating either an electron (particle) beam or an x-ray (photon) beam for use in the radiotherapy treatment of patients on a treatment couch 35. Other radiation treatment systems are capable of generating heavy ion particles such as protons. For purposes of the present discussion of FIG. 1, only x-ray irradiation will be discussed. However, it will be appreciated by those skilled in the art that the same principles apply to other (e.g. particle beam) systems.

Stand 10 supports a rotatable gantry 20 with a treatment head 30. Next to stand 10 there is arranged a control unit (not shown) that includes control circuitry for controlling the different modes of operation of the accelerator. A high voltage source is provided within the stand or in the gantry, to supply voltage to an electron gun (not shown) positioned on an accelerator guide located in the gantry 20. Electrons are emitted from the electron gun into the guide (not shown) where they are accelerated. A source supplies RF (microwave) power for the generation of an electric field within the waveguide. The electrons emitted from the electron gun are accelerated in the waveguide by the electric field, and exit the waveguide as a high energy electron beam, typically at megavoltage energies. The electron beam then strikes a suitable metal target, emitting high energy x-rays in the forward direction.

X-rays formed as described above are emitted from the target in the treatment head 30 in a divergent beam and reach a patient lying on the treatment couch 35. The beam may be shaped and directed in accordance with a treatment plan.

Although the above discussion relates to external beam therapy, it should be understood that the present invention is not so limited, and may, for example, be used to facilitate planning of other types of radiation therapy such as brachytherapy and proton therapy, and types of therapy that do not use radiation - such as ablation therapy and energy-based therapies in general.

An embodiment of the invention to be described may provide a system to help and guide the treatment path for a patient, helping to choose the most suitable care path.

The system may be used when a clinician has a cancer diagnosis case.

The embodiment provides a system (or hub) that handles interfacing between treatment planning tools (e.g. models, analytics tools, data visualisation tools ,etc.) and patient information (e.g. patient records). The treatment planning tools may be of known type - such as RapidPlan and Ethos, available from Varian. A front facing part of the system may be a graphical user interface (GUI) or dashboard that e.g. summarises the available information such as patient details, demographical details (how well the patient reflects other similar patients in the database) and gives access to insights provided by treatment planning tools.

The hub/system facilitates the collection of data and the generation of treatment predictions and planning.

Patients and different authorised users may interact with the hub, launch or request outputs from models, request data and statistics.

The system of FIG. 1 is an example of a treatment system than can be controlled according to a treatment plan for a particular patient.

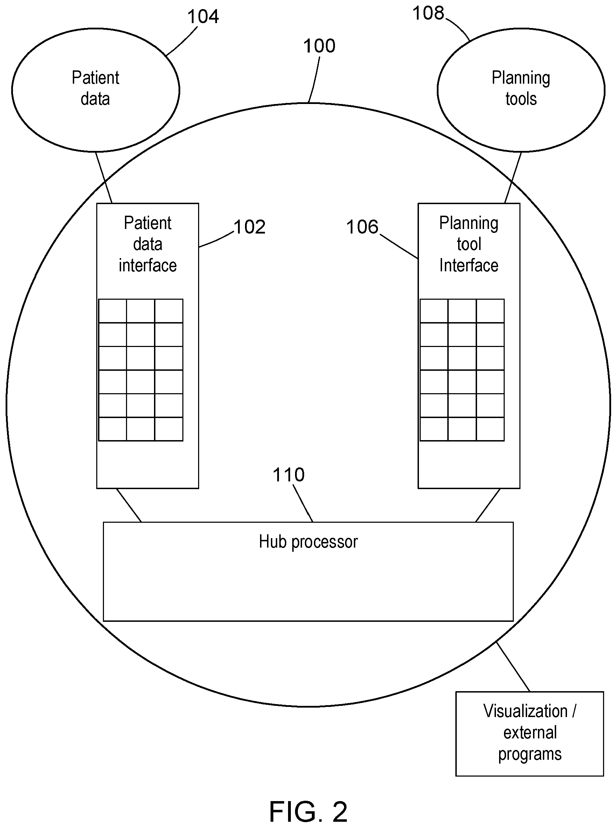

FIG. 2 shows an embodiment of a system (or hub) 100 for facilitating patient treatment planning. The system 100 includes a patient data interface 102 configured to communicate with patient data source 104 to send and receive data relating to a plurality of patients and to store data relating to the patients in an organised manner. The system 100 further includes a treatment planning tool interface 106 configured to communicate with treatment planning tools 108. The system 100 also includes a processor 110 coupled to the patient data interface 102 and the planning tool interface 106. The processor 110 may control a GUI.

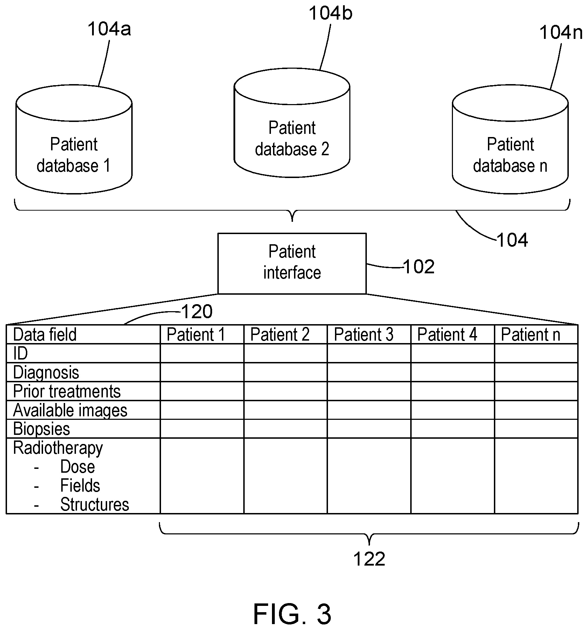

FIG. 3 shows the patient data source 104 and the patient data source interface 102 in more detail. The patient data source 104 may include one or more patient databases 104a, 104b, ..., 104n each storing information about one or more patients (patient records). Patient information may come from various different sources, such as being measured by different equipment and at different locations. Types of patient information include patient ID, patient diagnosis, patient prior treatments, patient available images, patient biopsies, multiomics information and patient prior radiotherapy treatment information such as dose, fields and structures. There are various multiomics that can be considered to affect personalised cancer treatments, for example genomics, proteomics, metablomics and epigenomics. The types of information available and stored for each patient may be different. For example, for one patient the diagnosis information available may be CT scan results and for another patient the diagnosis information available may be blood test results.

The information types for each patient may be stored in various formats. For example, two patients with a diagnosis of lung cancer may have information about the lung cancer recorded and stored in different formats - even if the diagnosis is the same - due to the diagnosis being made by a different physician, imaging device, etc.

Having different types of information for respective patients, or having the same type of information for different patients being held in different formats makes it challenging to use the patient information for treatment planning. Conventionally, treatment planning tools require specific types of patent information to be provided in a specific format, with the required types of information and format varying between treatment planning tools - and consequently patient information is often obtained and formatted specifically for a particular treatment planning tool, which is inefficient.

The patient data interface 102 is configured to maintain a plurality of patient interface data fields 120 relating to respective patient information types. The patient data interface 102 includes a memory that stores the patient interface data fields 120 for a plurality of different patients 122 (Patient 1, Patient 2, ..., Patient n).

For example, the patient information stored in respective patient interface data fields 120 may include: patient ID, patient diagnosis, patient prior treatments, patient available images, patient biopsies, multiomics information and patient prior radiotherapy treatment information such as dose, fields and structures. The patient information stored in each patient interface data field 120 has a predetermined format. The format is the same for each patient 122.

The patient data interface 102 is coupled to the patient data source 104 and receives patient information therefrom relating to the patients 122. As mentioned above, the patent information in the patient data source 104 may have various different (inconsistent) formats. The patient data source interface 102 converts the patient information received from the patient data source 104 into the predetermined format specified for each data field 120 and stores the converted data in the predetermined format in the data field 120. The patient data interface 102 therefore maintains a record for each patient 122 comprising ones of the data fields 120 derivable from the received patient information and in a consistent format for all the patients 122. The patient data source interface 102 data fields may be meta data fields.

The patient data interface 102 regularly communicates with the patient data source 104 to receive changes to the patient data or new patient data. The patient data interface 102 converts any changed or new patient information received from the patient data source 104 into the predetermined format from each relevant data field 120 and stores the converted data in the predetermined format in the data field 120.

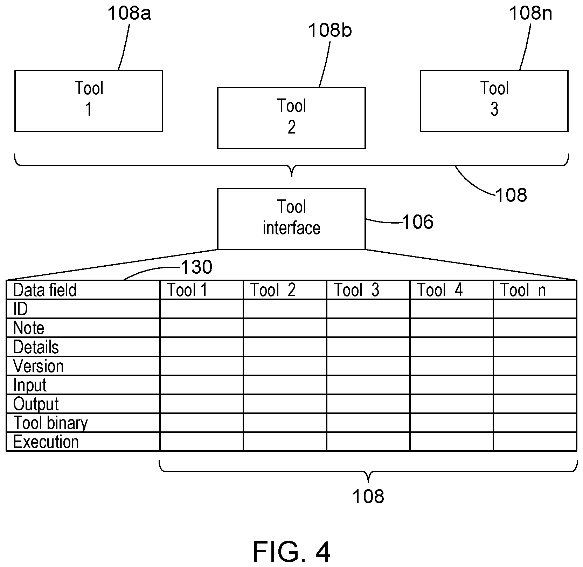

FIG. 4 shows the treatment planning tools 108 and the planning tool interface 106 in more detail. The treatment planning tool may be an analytics tool. The treatment planning tool may be a treatment planning model, a prediction model or descriptive model. The planning tools 108 may include one or more planning tools 108a, 108b, ..., 108n. Treatment planning information types required by respective planning tools 108a, 108b, ..., 108n may be different. The format of the treatment planning information required by each treatment planning tool 108a, 108b, ..., 108n may be different. Treatment planning information types output by respective planning tools 108a, 108b, ..., 108n may be different. The format of the treatment planning information output for each treatment planning tool 108a, 108b, ..., 108n may be different.

The planning tool interface 106 is configured to maintain a plurality of tool data fields 130 relating to tool characteristics and tool planning information. The planning tool interface 106 includes a memory that stores the tool interface data fields 130 for a plurality of different tools 108a, 108b, ..., 108n (Tool 1, Tool 2, ..., Tool n).

For example, the tool information stored in respective tool interface data fields 130 may include: tool ID/name, tool note, tool details, tool version, interface version, tool input, tool output, tool binary, tool execution, alternative launcher, associated software and serving method. The tool characteristic stored in each tool interface data field 130 has a predetermined format. The format is the same for each tool 132.

The planning tool interface 106 is coupled to the planning tools 108 and receives tool data therefrom relating to each of the tools 108a, 108b, ..., 108n. As mentioned above, the tool data output by the treatment planning tools 108a, 108b, ..., 108n may have various different formats. The planning tool interface 106 converts the tool data received from the tools 108 into the predetermined format from each data field 130. The planning tool interface 106 therefore maintains a record for each tool 132 comprising ones of the tool data fields 130 derivable from the received data relating to the tool and in a consistent format for all the tools 132. The tool data fields 130 may be meta data fields.

If a treatment planning tool 108a, 108b, ..., 108n changes, the data required by the planning tool 108a, 108b, ..., 108n may change. The planning tool interface 106 regularly communicates with tools 108a, 108b, ..., 108n to receive any changes and to update the planning tool interface 106 accordingly.

A treatment planning tool may be added. The planning tool interface 106 regularly communicates with tools to receive details of any new tools and to update the planning tool interface 106 accordingly.

The planning tool interface 106 is coupled to the planning tools 108a, 108b, ..., 108n and sends tool data thereto relating to the tools 108a, 108b, ..., 108n. The format of the data required by each planning tool 108a, 108b, ..., 108n may be different. The planning tool interface 106 converts the tool data from the predetermined format stored in each data field 130 into an appropriate format for the tool 108a, 108b, ..., 108n prior to sending the data to the tool.

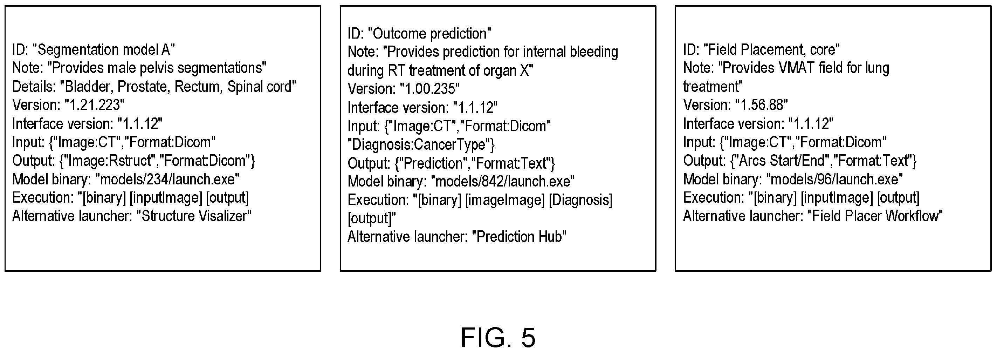

Each model in the model database has associated metadata that lists e.g. at least the name, version, data input requirements and serving method. Additionally, metadata can list interface scheme version for handling versioning during software lifecycle, alternative ways to serve the model (e.g., launch with a binary and show the result immediately in the GUI, or use external software to handle the model serving and visualization). FIG. 5 illustrates three examples of model metadata descriptions. The metadata allows for flexible description of models that can have a wide range of input and output descriptions. For the alternative launchers, the names would have to be mapped in processor 110 to existing workflows so that a launcher interface (e.g., a button in the GUI that executes an external application) could be provided to the user.

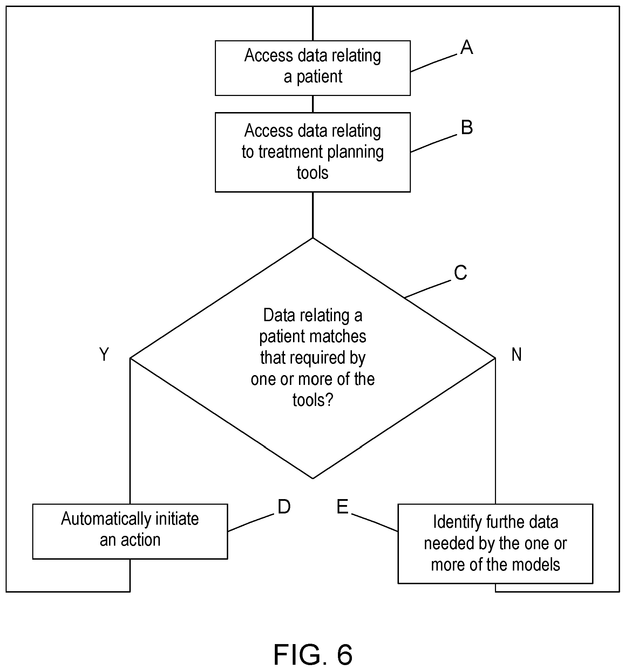

To facilitate patient treatment planning the processor 110 of the system 100 performs the steps shown in FIG. 6.

At step A the processor 110 accesses data in data fields 120 relating to a patient 122 from the patient data interface 102.

At step B the processor 110 accesses data required in relevant data fields 130 relating to the treatment planning tools 108a, 108b, ..., 108n, that indicate data needed for a planning tool to perform a planning function, from the planning tool interface 106.

At step C the processor 110 determines whether the data in data fields 120 relating to each patient 122 matches data (input data) required by one or more of the treatment planning tools 108a, 108b, ..., 108n in relevant data fields 130. The processor 110 is configured such that determining whether the data relating to a patient 122 matches that required by one or more of the treatment planning tools 108a, 108b, ..., 108n includes analysing data from the records in the planning tool interface 106 corresponding to the one or more of the tools 108a, 108b, ..., 108n. The processor 110 is configured such that determining whether the data relating to a patient 122 matches that required by one or more of the treatment planning tools 108a, 108b, ..., 108n includes analysing data from the records in data fields 120 in the patient data interface 102 corresponding to the patient 122.

If the data in data fields 120 relating a patient 122 matches that required in data fields 130 by one or more of the planning tools 108a, 108b, ..., 108n, the processor 110 automatically initiates an action at **step D.**

The action may include using the treatment planning tools 108a, 108b, ..., 108n to perform segmentation, beam placement or automatic radiotherapy planning based on the data relating to the patient. The action may include using the treatment planning tools 108a, 108b, ..., 108n to predict an outcome of a treatment planned by the treatment planning tools corresponding to the data relating to the patient 122, such as survivability and/or side effects.

The action may include executing the one or more of the treatment planning tools 108a, 108b, ..., 108n to produce treatment planning tool data output based on the data relating to the patient. The action may include prompting a user via the GUI prior to executing the one or more of the treatment planning tools 108a, 108b, ..., 108n to produce treatment planning tool data output corresponding to the data relating to the patient 122.

The action may include listing via the GUI the treatment planning tools 108a, 108b, ..., 108n ready for execution for the patient 122.

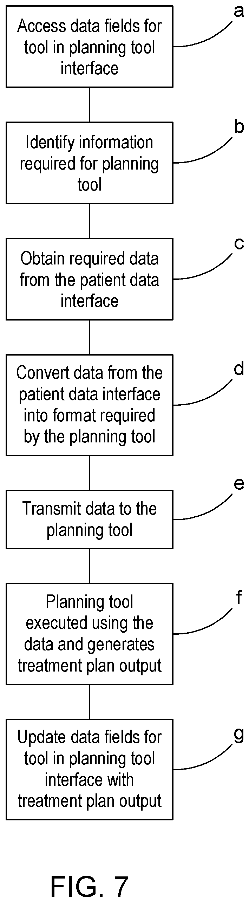

When it is desired to execute a treatment planning tool 108a, 108b, ..., 108n, the steps performed in the flow chart of FIG. 7 are performed. At **step a** the processor 110 accesses data fields for the tool 108a, 108b, ..., 108n in the planning tool interface 106. At **step b** the processor 110 identifies the data (input data) required for the tool 108a, 108b, ..., 108n using information accessed from the data fields 130 for that tool in the planning tool interface 106. The processor at **step c** 110 obtains the required data from the patient data interface 102. The processor 110 at **step d** converts the data from the patient data interface 102 into the format required by the tool 108a, 108b, ..., 108n and transmits the data at **step e** to the tool 108a, 108b, ..., 108n with an instruction for the tool 108a, 108b, ..., 108n to process the data. The tool 108a, 108b, ..., 108n then at **step f** performs the treatment planning and generates a treatment plan output. The treatment plan output is received by the processor 110 and information from the treatment plan output is extracted. The patient data fields 120 for the patient are updated at **step g** with the extracted information after it is converted into the relevant predetermined format used for that data type by the patient data source interface 102. That is, the data relating to the patient 122 is expanded or updated with new information following running of the planning tool 108a, 108b, ..., 108n and generation of the treatment plan output. The processor 110 may display via the GUI information from the treatment plan output. The process of FIG. 6 then returns to step A.

If the data relating a patient does not match that required by one or more of the treatment planning tools 108a, 108b, ..., 108n, the processor 110 may identify further data needed by one or more of the tools 108a, 108b, ..., 108n for the patient 122 at step E of FIG. 6. The processor 110 may indicate the further data needed by the one or more of the treatment planning tools 108a, 108b, ..., 108n for the patient 122. The processor 110 may indicate the further data needed by the one or more of the treatment planning tools 108a, 108b, ..., 108n for the patient in priority order. The processor 110 may be configured to automatically trigger processing by one of the treatment planning tools 108a, 108b, ..., 108n to obtain the further data needed by the one or more of the treatment planning tools 108a, 108b, ..., 108n for the patient 122. The process of FIG. 6 then returns to step A.

At D or E of FIG. 6 patient data may have been added to the data fields 120 of the patient data interface 102 (or, as mentioned above, new or changed data for the patient 122 may be received from the patient data source 104). When the process iterates the patient data accessed at step A of FIG. 6 of FIG. 6 includes any new patient data.

As mentioned above, the data 130 relating to the tools in the tool interface 106 may also be updated when a tool is changed or added. The tool data 130 accessed at step B includes any new patient data.

At step C of FIG. 6, when the processor 110 determines whether the data relating to each patient matches that required by one or more of the treatment planning tools 108a, 108b, ..., 108n about which data are stored in the tool data source 108, additional matches may be found due to the updated patient and tool data. For example, the results of execution of one treatment planning tool 108a, 108b, ..., 108n may provide new information that allows another tool to execute.

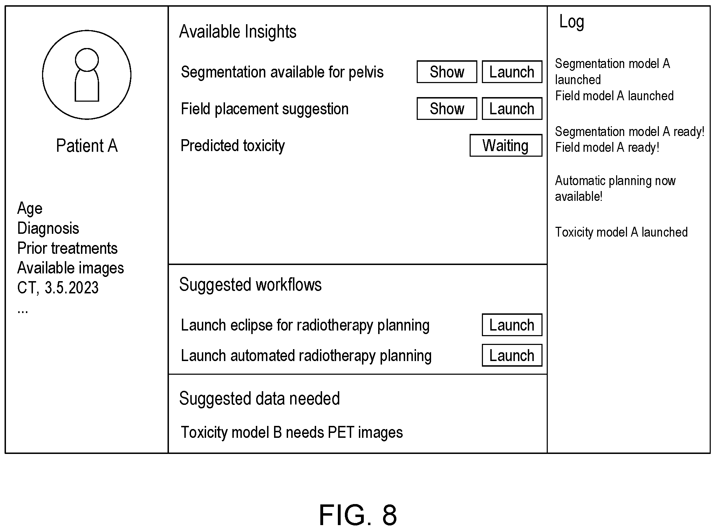

FIG. 8 shows an example of the information visualisation that may be provided by the GUI. It should be understood that more, different or fewer visualisation elements may be shown.

The basic interaction with the system 100 may take place through a dashboard-like access provided by the GUI. There may be different workspaces in the GUI.

The system may include an Al-powered chat bot/large language model. The chat bot/large language model may be configured to respond to natural language queries about patients or patient cohorts in the system 100, such as which treatment path is likely to be the most efficient for the patient. The system may be configured to create human-understandable summaries of the patient based on all of the information in the interfaces 102 and 106. This information amount is typically so big, that it is difficult to go through it all manually.

The system 100 may be configured to create and display on the GUI patient treatment information for a patient including at least one of patient characteristics, treatment planning tool output for the patient, suggested further treatment planning workflows and suggested further data relating to the patient required.

The embodiment may provide insights in a step-wise manner as soon as new information becomes available and/or the treatment progresses. For example, if some imaging/biopsy is needed at any point, the tools may request it. Processes such as segmentation, beam placement, automatic radiotherapy planning, etc., may be started manually via the GUI or automatically in the background if the input data is already available. Any missing data may be requested if the system evaluates some process to be important.

The system 100 may include means for authenticating users for selectively making available output of the system in dependence upon a user's access rights.

### Computer system

Any of the procedures, steps or methods mentioned herein may utilise a computer system or any suitable number of computer subsystems. Examples of such subsystems are shown in FIG. 8 in computer system 1800. In some embodiments, a computer system includes a single computer apparatus, where the subsystems can be the components of the computer apparatus. In other embodiments, a computer system can include multiple computer apparatuses, each being a subsystem, with internal components.

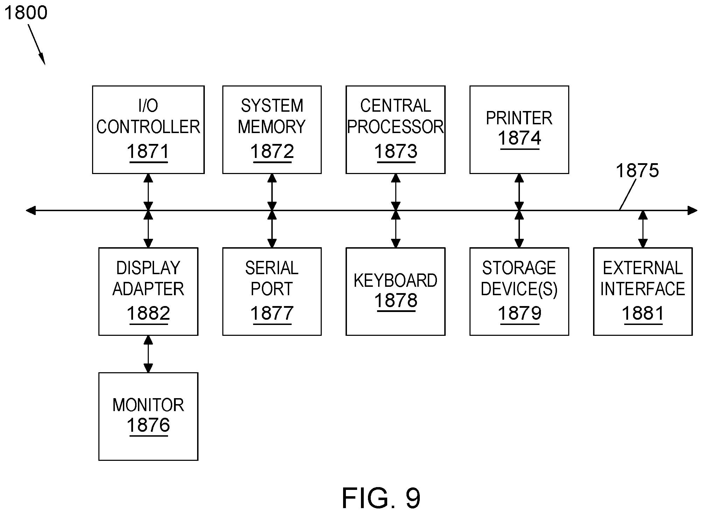

The subsystems shown in FIG. 9 are interconnected via a system bus 1875. Additional subsystems such as a printer 1874, keyboard 1878, storage device(s) 1879, monitor 1876, which is coupled to display adapter 1882, and others are shown. Peripherals and input/output (I/O) devices, which couple to I/O controller 1871, can be connected to the computer system by any number of means known in the art, such as serial port 1877. For example, serial port 1877 or external interface 1881 (e.g. Ethernet, Wi-Fi, etc.) can be used to connect computer system 1800 to a wide area network such as the Internet, a mouse input device, or a scanner. The interconnection via system bus 1875 allows the central processor 1873 to communicate with each subsystem and to control the execution of instructions from system memory 1872 or the storage device(s) 1879 (e.g., a fixed disk, such as a hard drive or optical disk), as well as the exchange of information between subsystems. The system memory 1872 and/or the storage device(s) 1879 may embody a computer readable medium. Any of the data mentioned herein can be output from one component to another component and can be output to the user.

A computer system can include a plurality of the same components or subsystems, e.g., connected together by external interface 1881 or by an internal interface. In some embodiments, computer systems, subsystems, or apparatuses can communicate over a network. In such instances, one computer can be considered a client and another computer a server, where each can be part of a same computer system. A client and a server can each include multiple systems, subsystems, or components.

It should be understood that any of the embodiments of the present invention can be implemented in the form of control logic using hardware (e.g. an application specific integrated circuit or field programmable gate array) and/or using computer software with a generally programmable processor in a modular or integrated manner. As used herein, a processor includes a multi-core processor on a same integrated chip, or multiple processing units on a single circuit board. Based on the disclosure and teachings provided herein, a person of ordinary skill in the art will know and appreciate other ways and/or methods to implement embodiments of the present invention using hardware and a combination of hardware and software.

Any of the computer implemented methods described in this application may be implemented as software code to be executed by a processor using any suitable computer language such as, for example, Java, C++ or Perl using, for example, conventional or object-oriented techniques. The software code may be stored as a series of instructions or commands on a computer readable medium for storage and/or transmission; suitable media include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium according to an embodiment of the present invention may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the procedures, steps or methods described herein may be totally or partially performed with a computer system including one or more processors, which can be configured to perform the steps of the methods. Thus, embodiments can be directed to computer systems configured to perform the steps of any of the methods described herein, potentially with different components performing a respective step or a respective group of steps. Although presented as numbered steps, steps of methods herein can be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

Those skilled in the art will recognise that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A system for facilitating patient treatment planning, the system including:
a first interface (102) configured to communicate with a patient data source (104) to receive data relating to a patient;
a second interface (106) configured to communicate with a treatment planning tool data source to receive data relating to a plurality of treatment planning tools (108); and
a processor (110) coupled to the first interface (102) and the second interface (106), and configured to determine whether the data relating to the patient matches that required by one or more of the treatment planning tools (108) and, if the data relating a patient matches that required by one or more of the tools (108), to automatically initiate an action.

2. The system of claim 1, wherein the action includes:
using the treatment planning tools (108) to perform segmentation, beam placement or automatic radiotherapy planning corresponding to the data relating to the patient; or
using the treatment planning tools (108) to predict an outcome of a treatment planned by the treatment planning tools (108) corresponding to the data relating to the patient, such as survivability and/or side effects.

3. The system of claim 1 or 2, wherein the treatment planning tool (108) comprises an analytics tool, a treatment planning model, a prediction model or descriptive model.

4. The system of one of claims 1 to 3, wherein the second interface (106) is configured to maintain a plurality of second interface data fields (130) relating to respective treatment planning tool (108) characteristics, each second interface data field (130) having a predetermined format, and to maintain a record for each treatment planning tool (108) comprising ones of the second interface data fields (130) derivable from the received data relating the treatment planning tools (108).

5. The system of claim 4, wherein the second interface data fields (130) include one or more of name, version, data input requirements, treatment planning tool data output type and serving method.

6. The system of claim 4 or 5, wherein the processor (110) is configured such that determining whether the data relating to the patient matches that required by one or more of the treatment planning tools (108) includes analysing data from the records in the second interface (106) corresponding to the one or more of the treatment planning tools (108).

7. The system of any one of claims 1 to 6, wherein the action includes:
executing the one or more of the treatment planning tools (108) to produce treatment planning tool data output corresponding to the data relating to the patient;
prompting a user prior to executing the one or more of the treatment planning tools (108) to produce treatment planning tool data output corresponding to the data relating to the patient; and/or
listing the treatment planning tools (108) ready for execution.

8. The system of one of claims 1 to 7, wherein the first interface (102) is configured to maintain a plurality of first interface data fields (120) relating to respective patient characteristics, each first interface data field (120) having a predetermined format, and to maintain a record for each patient comprising ones of the first interface data fields (120) derivable from the received data relating to the patient.

9. The system of claim 8, wherein the first interface data fields (120) include one or more of patient information, results from health treatments, patient images and multiomics information.

10. The system of claim 8 or 9, wherein the processor (110) is configured such that determining whether the data relating to a patient matches that required by one or more of the treatment planning tools (108) includes analysing data from the records in the first interface (102) corresponding to the patient.

11. The system of any one of claims 1 to 10, wherein the processor (110) is configured to determine whether the data relating to a patient does not match that required by one or more of the treatment planning tools (108) and, if the data relating a patient does not match that required by one or more of the treatment planning tools (108), to identify further data needed by the one or more of the treatment planning tools (108).

12. The system of claim 11, wherein the processor (110) is configured to automatically trigger processing by one of the treatment planning tools (108) to obtain the further data needed by the one or more of the treatment planning tools (108).

13. The system of any one of claims 1 to 12, wherein the processor (108) is configured to receive treatment planning tool output data from one of the treatment planning tools (108) and to determine whether the treatment planning tool output data therefrom matches that required by one or more of the other treatment planning tools (108) and, if the treatment planning tool output data matches that required by one or more of the other the treatment planning tools (108), to automatically execute a further action.

14. A method of facilitating patient treatment planning, the method including:
providing a first interface (102) configured to communicate with a patient data source (104) to receive data relating to a patient;
providing a second interface (106) configured to communicate with a treatment planning tool data source to receive data relating to a plurality of treatment planning tools (108); and
using a processor (110) coupled to the first interface (102) and the second interface (106) to determine whether the data relating to the patient matches that required by one or more of the treatment planning tools (108) and, if the data relating a patient matches that required by one or more of the tools (108), automatically initiating an action.

15. A system for facilitating patient treatment planning, the system including:
means for communicating with a patient data source (104) to receive data relating to a patient;
means for communicating with a treatment planning tool data source to receive data relating to a plurality of treatment planning tools (108);
processing means coupled to the first interface (102) and the second interface (106) for determining whether the data relating to the patient matches that required by one or more of the treatment planning tools (108) and, if the data relating a patient matches that required by one or more of the tools (108), for automatically initiating an action.
